# EUROPEAN PATENT SPECIFICATION

(11) **EP 1 104 747 B1**
(45) Date of publication and mention of the grant of the patent: **30.07.2003**
(21) Application number: 00204232.3
(22) Date of filing: 29.11.2000
(51) Int. Cl.: C07C 2/20, C07C 5/03

(54) **Process for the preparation of oligomers of internal olefins**
Verfahren zur Herstellung von Oligomeren von internen Olefinen
Procédé pour la préparation d'oligomères d'oléfines internes

(30) Priority: 03.12.1999 IT MI992539
(43) Date of publication of application: 06.06.2001
(73) Proprietor: SASOL ITALY S.p.A., 90144 Palermo (IT); ENITECNOLOGIE S.p.A., 20097 S. Donato Milanese (Milano) (IT)
(72) Inventor: Marcotullio, Armando, 20097 San Donato Milanese(Milan) (IT); Borgarello, Enrico, 26833 Merlino(Lodi) (IT); Zatta, Agostino, 20098 San Giuliano Milanese(Milan) (IT); Radici, Pierino, 22078 Turate(Como) (IT); Cozzi, Pierluigi, 20014 Nerviano(Milan) (IT)
(74) Representative: De Gregori, Antonella

(56) References cited:
- EP-A- 0 136 377
- EP-A- 0 776 960

## Description

The present invention relates to a process for the preparation of oligomers from internal C₁₃-C₁₄ olefins and their use for the synthesis of bases for synthetic or semi-synthetic lubricants.

The possibility of obtaining bases for lubricating oils by oligomerization and subsequent hydrogenation of internal olefins in the presence of suitable catalysts, is described in literature.

For example, the patent EP-68554 describes the oligomerization of internal olefins, preferably internal olefins having from 15 to 18 carbon atoms.

The above oligomerization is carried out in the presence of a catalytic system consisting of a supported inorganic acid, or its anhydride, and gaseous BF₃, at a temperature preferably ranging from 80 to 100°C. As can be observed from mass spectrometry and bromometric titrations, the oligomers thus obtained generally have a double bond for each molecule.

The oligomerization usually produces a mixture of dimers and trimers, higher oligomers generally being less than 5-10%. The unsaturated oligomers are subsequently hydrogenated in the presence of catalysts well known to experts in the field. The products thus obtained, free of any possible light-products, are called PIO (poly internal olefins).

The main use of the above PIOs, particularly those deriving from compositions prevalently consisting of internal C₁₅-C₁₈ olefins, is as a base for synthetic or semi-synthetic lubricating oils.

A process has now been found which, with the same viscosity at high temperatures, allows an improvement in the viscosity at low temperatures of the above PIOs starting from internal C₁₃-C₁₄ olefins.

In accordance with this, an objective of the present invention relates to a process for the production of oligomers starting from internal C₁₃-C₁₄ olefins which comprises contacting said olefins with an acid catalyst, in the presence of gaseous BF₃, at a temperature of from 5 to 35°C and with a weight ratio BF₃/100 g of olefins ranging from 1.0 to 3.0.

The term internal olefins refers to olefins in which the double bond is not only present in position 1,2 (like alpha-olefins) but is statistically distributed along the whole chain.

Said olefins are preferably obtained with the process called PACOL-OLEX, by dehydrogenation of n-paraffins.

In particular, in the oligomerization reaction inorganic acids, preferably selected from sulfuric acid, sulfurous acid, phosphoric acid and/or their anhydrides, are used as catalysts.

The ratio between inorganic acid and olefins ranges from 0.2 to 0.6, preferably from 0.3 to 0.5, grams of acid per 100 grams of olefins.

In a preferred embodiment the weight ratio BF₃/olefins ranges from 1.5 to 2.7 grams of BF₃ per 100 grams of olefins.

At the end of the oligomerization, the reaction raw product is neutralized and washed with distilled water at 60°C, under pH control.

The reaction products consist of oligomers of the starting olefins, prevalently dimers, trimers and tetramers with small quantities of pentamers and hexamers, as shown by gaschromatographic analysis coupled with mass spectrometry.

For use in the field of synthetic lubricating oils, the oligomers obtained must be subjected to a hydrogenation process effected in the presence of suitable catalysts and under the usual conditions, in particular of pressure and temperature, for the hydrogenation of olefins.

The hydrogenation reaction is preferably carried out in the presence of catalysts based on Pd or Ni, as such or supported, at a temperature ranging from 150 to 240°C and a hydrogen pressure ranging from 0,98 to 3,92 MPa (10 to 40 Kg/cm²).

The characteristics of the oligomers do not vary in the hydrogenation process.

According to the oligomerization process described above, a conversion of the starting olefins ranging from 85 to 95% is usually obtained; an elimination step of the light fractions is therefore necessary, generally by distillation.

This step can be carried out at the end of the oligomerization reaction or, preferably, after the subsequent hydrogenation reaction.

Both physico-chemical and technological characterizations for use in the field of synthetic lubricants are effected on the end-products.

The kinematic viscosity at 40 and 100°C is determined according to the method ASTM-D445.

The viscosity at -30°C and the Pour Point are determined according to the methods ASTM-D2602 and ASTM-97, respectively.

The weight loss upon evaporation (Noack) is determined according to the method ASTM D-972 (DIN 51581).

The bases for lubricating oils thus prepared can be used as main component of synthetic lubricants or mixed with conventional mineral bases.

The mineral bases can be of the paraffinic, naphthenic or mixed paraffinic-naphthenic type, distillates or distillation residues or synthesis products.

In the case of formulations of lubricants for engines, hydrocarbon fractions essentially consisting of distillates of the paraffinic type are preferable.

Typical synthetic lubricant bases which can be optionally mixed with the oligomers obtained with the process of the present invention, are esters of aliphatic monocarboxylic acids with polyhydroxylic alcohols such as trimethylol propane and pentaerythritol; esters of diacids with monofunctional alcohols, synthetic hydrocarbons, polyglycols, thiols, siliconic fluids, polyphenyl ethers and thioethers.

The compositions containing the oligomers obtained in the present invention may comprise conventional additives.

The selection of these additives to be included in the end-oil and the relative quantities depend on the use and performances desired.

For example, additives for improving the viscosity index and pour point depressant are contained in the end-oil in a quantity ranging from 0.01 to 15% by weight; detergents-dispersing agents (typical examples of which are succinimide derivatives) in a quantity of 0.1 to 15% by weight; corrosion and wear inhibitors in a quantity of 0.01 to 3% by weight; antifoam agents from 10 to 100 ppm.

Many other additives with different functions can be used in preparing the end-formulates. Some of these are listed in patents US 3,864,270, US 4,169,799, US 4,194,981 and US 4,253,980.

The following examples are provided for a better understanding of the present invention.

### 1. COMPOSITION OF n-OLEFINS

The substrates subjected to oligomerization and hydrogenation are:
(A) a C₁₃-C₁₄ mixture having the following weight composition:
   - C₁₃ n-olefins 4.81%
   - C₁₄ n-olefins 82.85%
   - C₁₃ n-paraffins 0.98%
   - C₁₄ n-paraffins 3.82%
   - diolefins 4.90%
   - aromatics 2.30%
   - others complement to 100
(B) a C₁₅-C₁₇ mixture having the following weight composition:
   - C₁₅ n-olefins 70.68%
   - C₁₆ n-olefins 19.55%
   - C₁₇ n-olefins 2.45%
   - C₁₅ n-paraffins 2.81%
   - C₁₆ n-paraffins 1.07%
   - C₁₇ n-paraffins 0.16%
   - aromatics + diolefins 3.28%
(C) a C₁₄-C₁₉ mixture having the following weight composition:
   - C₁₄ n-olefins 3.38%
   - C₁₅ n-olefins 22.03%
   - C₁₆ n-olefins 22.47%
   - C₁₇ n-olefins 22.12%
   - C₁₈ n-olefins 16.98%
   - C₁₉ n-olefins 2.60%
   - n-paraffins + others 10.42%

### 2. SYNTHESIS OF THE BASES

The synthesis essentially consists of an oligomerization step of the olefins, followed by neutralization and washing of the BF₃·acid complex, a hydrogenation step of the oligomers thus obtained and finally a stripping step of the light products.

### 2.1 OLIGOMERIZATION

A 1 litre AISI 316 Brignole^{R} autoclave is used for the oligomerization, equipped with a magnetic drag stirrer, 4 valves of which one is plunged, a thermometric well, a thermocouple, a digital indicator for the temperature measurement and a manometer with a scale-end of 2,35 MPa (24 Kg/cm²).

The oligomerization is carried out by charging the olefin, H₃PO₄ acid and P₂O₅ into the autoclave with the top open. A pressure-seal test is carried out with anhydrous nitrogen and flushing is effected 5 times at 0,49 MPa (5 Kg/cm²), always with nitrogen, alternating the movement of the stirrer.

The nitrogen is subsequently degassed and BF3 is fed from a previously weighed cylinder. The stirring is started (760 rpm) and the autoclave is brought to the pre-selected temperature.

The reaction heat is absorbed by circulating aqueduct water (17°C) or cryogenic fluid (cooled alcohol) in the specific coil of the autoclave thus obtaining a control of the operating temperature.

All the tests, comprising the comparative ones, were effected using from 1.36 to 2.8 grams of BF₃ per 100 grams of olefins fed, from 0.41 to 0.47 grams of H₃PO₄ per 100 grams of olefins and a constant P₂O₅/olefin ratio (0.032 g of P₂O₅ per 100 g of olefins).

After 3 hours of reaction, the BF₃ is degassed and is sent to specific NaOH + Ca(OH)₂ traps; the autoclave is repeatedly flushed with nitrogen and the content of the autoclave is discharged into a beaker which is weighed. The oligomers are neutralized with an aqueous solution of soda at 60°C and under pH control, and are subsequently washed with distilled water with an oil/water ratio of 1.

The neutralized product is transferred to a 2-litre glass separator funnel and separated into two oil/water phases. The resulting oil phase (olefinic oligomers) is charged into an autoclave for hydrogenation.

### 2.2 HYDROGENATION

A 1 litre Hastelloy B Engineers autoclave is used for the hydrogenation of the oligomers, equipped with a magnetic drag stirrer, 4 valves, a thermometric well, a thermocouple, a digital indicator for the temperature measurement, a burst disk calibrated at 5 MPa (50 bars) and a manometer with a scale-end of 3,92 MPa (40 Kg/cm²).

The neutralized olefinic oligomers and catalyst (Pd/C at 10% of Pd) are charged into the autoclave with the top open, in a ratio of 4% with respect to the charge to be hydrogenated.

A pressure-seal test is carried out with anhydrous nitrogen at 3,92 MPa (40 Kg/cm²) and a flushing is effected 7 times at 0,49 MPa (5 Kg/cm²), again with nitrogen, alternating the movement of the stirrer.

The autoclave is heated to 150°C and is pressurized with hydrogen at 1,96-3,92 MPa (20-40 Kg/cm²). The hydrogen is reintegrated by a specific valve situated on the top of the autoclave, when the pressure decreases from 3,92 to 1,96 MPa (40 to 20 Kg/cm²).

The reaction (exothermic) is carried out with stirring at 1,500 rpm, the temperature being maintained within values ranging from 170-200°C for 7-8 hours.

At the end of the reaction, the hydrogen is degassed, the autoclave is flushed 3 times with nitrogen at 0,49 MPa (5 Kg/cm²) and the product is discharged and then weighed.

The oligomers thus hydrogenated are separated from the catalyst by filtration and are then distilled.

A 2,54 cm (1-inch) adiabatic column (Oldershaw type) with 5 plates is used for the stripping, i.e. the distillation of the light products formed during the reaction and non-oligomerized products.

The operation is carried out at reduced pressure (about 0,66-1,33 MPa (0.5-1 torr)) and with a final temperature at the top of about 170-180°C until complete removal of the heads, verified by gaschromatographic analysis on both the head and tail residues in the boiler.

### 3. RHEOLOGICAL CHARACTERIZATION OF THE END-OIL

Gaschromatographic analyses are effected on the weighed distillate and boiler residue and the residue is subjected to rheological characterization.

The rheological characterization of the bases is effected at 40 and 100°C using a series of capillaries of the Cannon Fenske type and a thermostatic bath with a control to the hundredth centigrade degree.

For the Pour Point and viscosity measurements at -30°C, a rotating rheometer with strain control, model RMS800 of Rheometrics, is used. The strain rate range applied is 0.1-1,000 sec⁻¹ and the relative viscosity value indicated relates to the flow curve value at 100 sec⁻¹.

A frequency of 1 Hz, a strain amplitude of 0.2% and a temperature increase of 1°C/minute are used for the Pour Point measurements.

The weight loss upon evaporation (Noack) is equal to 11%.

The results of the tests and analyses are indicated in Table 1.

Examples 7, 8, 9 and 10 are comparative examples as they are carried out at temperatures higher than 60°C.

Examples 9 and 10 were carried out using a mixture of internal C₁₅-C₁₇ olefins and a C₁₄-C₁₉ mixture, respectively.

**TABLE 1**

| Ex. | BF₃/olefin (% weight) | T°C | P. BF₃ (Kg/cm²) MPa | % yield end oil | Visc. (cSt) (40°C) | Visc. (cSt) (100°C) | Visc. (cp) (-30°C) | P.P. °C |
|---|---|---|---|---|---|---|---|---|
| 1 | 1.56 | 30 | (5.6) 0.55 | 85.4 | 28.80 | 5.39 | 2570 | -72 |
| 2 | 1.84 | 30 | (7.5) 0.74 | 85.0 | 30.71 | 5.64 | 2810 | -72 |
| 3 | 2.50 | 30 | (10.0) 0.98 | 85.3 | 35.37 | 6.20 | 3520 | -71 |
| 4 | 2.23 | 30 | (9.1) 0.89 | 85.0 | 34.20 | 6.05 | 3430 | -72 |
| 5 | 2.67 | 8 | (9.7) 0.86 | 92.9 | 37.86 | 6.52 | 4100 | -67 |
| 6 | 1.43 | 8 | (5.0) 0.49 | 87.8 | 31.45 | 5.81 | 2700 | -65 |
| 7 | 2.56 | 65 | (11.5) 1.02 | 83.5 | 30.90 | 5.62 | 2670 | -76 |
| 8 | 1.70 | 65 | (7.7) 0.75 | 81.7 | 24.75 | 4.80 | 1880 | -79 |
| 9 | 1.68 | 65 | (6.0) 0.59 | 85.0 | 29.53 | 5.62 | 2495 | -51 |
| 10 | 1.15 | 65 | (4.0) 0.39 | 84.8 | 33.38 | 6.15 | 3175 | -27 |

The results of the table clearly show the advantages, in terms of viscosity at -30°C, pour point and oil yield, obtained with the process of the present invention.

## Claims

1. A process for the production of oligomers useful as bases for synthetic lubricants starting from internal C₁₃-C₁₄ olefins, which comprises:
(a) contacting said olefins with an acid catalyst in the presence of gaseous BF3 at a temperature of from 5 to 35°C;
(b) neutralizing and washing the reaction raw product;
(c) hydrogenating the oligomers thus obtained and, finally;
(d) removing the light products from the end-oil obtained in (c).

2. The process according to claim 1, wherein the catalyst is an inorganic acid selected from the group consisting of sulfuric acid, sulfurous acid, phosphoric acid and/or their anhydrides.

3. The process according to claim 1, wherein the ratio between the catalyst and olefin ranges from 0.2 to 0.6 grams of acid catalyst per 100 grams of olefins.

4. The process according to claim 3, wherein the ratio between the catalyst and olefin ranges from 0.3 to 0.5 grams of acid catalyst per 100 grams of olefins.

5. The process according to claim 1, wherein the weight ratio BF₃/100 g of olefins ranges from 1.0 to 3.0.

6. The process according to claim 5, wherein the weight ratio BF₃/100 g of olefins ranges from 1.5 to 2.7.

7. The process according to claim 1, wherein the hydrogenation reaction is carried out in the presence of catalysts based on Pd or Ni, as such or supported, at a temperature ranging from 150 to 240°C and a hydrogen pressure ranging from 0,98 to 3,92 MPa (10 to 40 Kg/cm²).

## Patentansprüche

1. Verfahren zur Herstellung von Oligomeren, die als Grundstoffe zur Synthese von synthetischen Gleitmitteln brauchbar sind, ausgehend von internen C₁₃-C₁₄-Olefinen, welches umfaßt:
(a) Inkontaktbringen dieser Olefine mit einem Säurekatalysator in Anwesenheit von gasförmigem BF₃ bei einer Temperatur von 5 bis 35°C;
(b) Neutralisieren und Waschen des Reaktions-Rohproduktes;
(c) Hydrieren der so erhaltenen Oligomeren; und abschließend
(d) Entfernen der leichten Produkte aus dem in (c) erhaltenen Endöl.

2. Verfahren nach Anspruch 1, bei welchem der Katalysator eine anorganische Säure, ausgewählt aus der aus Schwefelsäure, schwefliger Säure, Phosphorsäure und/oder deren Anhydriden bestehenden Gruppe, ist.

3. Verfahren nach Anspruch 1, bei welchem das Verhältnis zwischen dem Katalysator und dem Olefin von 0,2 bis 0,6 Gramm von Säurekatalysator pro 100 Gramm von Olefinen reicht.

4. Verfahren nach Anspruch 3, bei welchem das Verhältnis zwischen dem Katalysator und dem Olefin von 0,3 bis 0,5 Gramm von Säurekatalysator pro 100 Gramm von Olefinen reicht.

5. Verfahren nach Anspruch 1, bei welchem das Gewichtsverhältnis BF₃/100 g Olefine von 1,0 bis 3,0 reicht.

6. Verfahren nach Anspruch 5, bei welchem das Gewichtsverhältnis BF₃/100 g Olefine von 1,5 bis 2,7 reicht.

7. Verfahren nach Anspruch 1, bei welchem die Hydrierungsreaktion in Anwesenheit von auf Pd oder Ni basierenden Katalysatoren, als solche oder getragen, bei einer Temperatur im Bereich von 150 bis 240°C und bei einem Wasserstoffdruck im Bereich von 0,98 bis 3,92 MPa (10 bis 40 kg/cm² durchgeführt wird.

## Revendications

1. Procédé pour la production d'oligomères utiles comme bases pour des lubrifiants synthétiques en partant d'oléfines internes en C₁₃-C₁₄, qui comprend les opérations consistant à :
a) mettre en contact lesdites oléfines avec un catalyseur acide en présence de BF₃ gazeux à une température allant de 5 à 35°C;
b) neutraliser et laver le produit brut de réaction;
c) hydrogéner les oligomères ainsi obtenus et, pour finir,
d) retirer les produits légers de l'huile finale obtenue en c).

2. Procédé selon la revendication 1, dans lequel le catalyseur est un acide inorganique choisi dans le groupe consistant en acide sulfurique, acide sulfureux, acide phosphorique et/ou leurs anhydrides.

3. Procédé selon la revendication 1, dans lequel le rapport entre le catalyseur et l'oléfine se situe dans une plage allant de 0,2 à 0,6 gramme de catalyseur acide pour 100 grammes d'oléfines.

4. Procédé selon la revendication 3, dans lequel le rapport entre le catalyseur et l'oléfine se situe dans une plage allant de 0,3 à 0,5 gramme de catalyseur acide pour 100 grammes d'oléfines.

5. Procédé selon la revendication 1, dans lequel le rapport pondéral BF₃/100 g d'oléfines se situe dans une plage allant de 1,0 à 3,0.

6. Procédé selon la revendication 5, dans lequel le rapport pondéral BF₃/100 g d'oléfines se situe dans une plage allant de 1,5 à 2,7.

7. Procédé selon la revendication 1, dans lequel la réaction d'hydrogénation est conduite en présence de catalyseurs à base de Pd ou de Ni, en tant que tels ou supportés, à une température dans une plage allant de 150 à 240°C et une pression d'hydrogène variant entre 0,98 à 3,92 Mpa (10 à 40 kg/cm²).
